# EUROPEAN PATENT SPECIFICATION

(11) **EP 0 745 603 B1**
(45) Date of publication and mention of the grant of the patent: **02.12.1998**
(21) Application number: 96103909.6
(22) Date of filing: 13.03.1996
(51) Int. Cl.: C07D 501/04

(54) **Process for p-nitrobenzyl ester cleavage in cephalosporin**
Verfahren zur Spaltung von p-Nitrobenzylester in Cephalosporin
Procédé de clivage de p-nitrobenzyl-ester de céphalosporine

(30) Priority: 30.05.1995 US 453911
(43) Date of publication of application: 04.12.1996
(73) Proprietor: Ranbaxy Laboratories Limited, New Delhi 110020 (IN)
(72) Inventor: Khanna, Jag Mohan, New Delhi 110 049 (IN); Kumar, Yatendra, New Delhi 110 048 (IN); Arora, Rakesh Kumar, New Delhi 110 059 (IN); Tiwari, Neera, New Delhi 110 016 (IN); Singh, ShailendarKumar, Kalkaji Extention, New Delhi (IN)
(74) Representative: Cohausz & Florack

(56) References cited:
- US-A- 3 781 282
- HETEROCYCLES (HTCYAM,03855414);93; VOL.36 (8); PP.1729-34, LEDERLE (JAPAN) LTD.;CHEM. FORMUL. LAB.; SHIKI; 353; JAPAN (JP), XP002011714 KUMAGAI T ET AL: "Mild and chemoselective cleavage of p-nitrobenzyl esters and p-nitrobenzyloxycarbonylamines with zinc dust"
- "Encyclopedia of Reagents for organic synthesis" 1995 , JOHN WILEY & SONS , CHICHESTER, ENGLAND XP002011715 pages 144-153, 2871-2873, 3191-3197, 4888-4891

## Description

The invention relates to a novel method of removing p-nitrobenzyl groups from esters of cephalosporin. More particularly, p-nitrobenzyl esters of cephalosporins are reductively cleaved with iron and hydrochloric acid in a mixture of water and organic solvent to produce the corresponding cephalosporin carboxylic acids.

### BACKGROUND OF THE INVENTION

p-Nitrobenzyl esters of various cephalosporins are well known stable crystalline compounds and their cleavage by Zn/HCl (US 3,781,282), Zn/AcOH (US 3,925,372), Zn/thiophenol (GB 1,582,960), sodium dithionite (US 3,799,924) and Pd/C (JACS 96, 4986, 1974) have been recently described. Heterocycles 36, 1729-1734, 1993 discloses the use of zinc in a THF/phosphate buffer solution for the removal of p-nitrobenzyl groups from 1β-methylcarbapenem esters. However, such methods, especially Zn/HCl for the cleavage of p-nitrobenzyl esters, result in the concomitant production of an insoluble material which complicates the isolation and recovery of the desired cephalosporin carboxylic acids. It is accordingly an objective of the present invention to provide an improved process for converting p-nitrobenzyl ester of cephalosporin to the corresponding cephalosporin carboxylic acid using Fe, Al or Sn in combination with HCl in high yield and quality. This novel method has not been found so far in the literature.

### SUMMARY AND DETAILED DESCRIPTION OF PREFERRED EMBODIMENTS

In accordance with the present invention, a process for converting a cephalosporin p-nitrobenzyl ester represented by Formula I 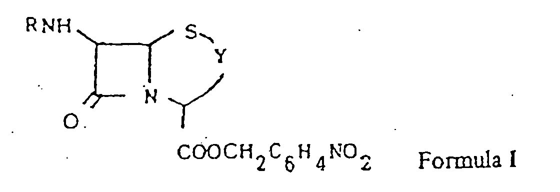 to a corresponding cephalosporin free acid represented by Formula II 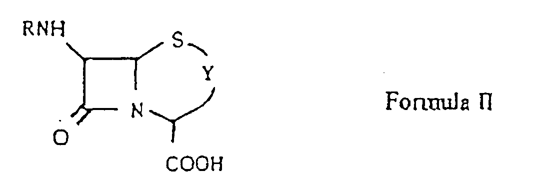 wherein R is hydrogen phenylacetyl, phenoxyacetyl or a group represented by Formula III 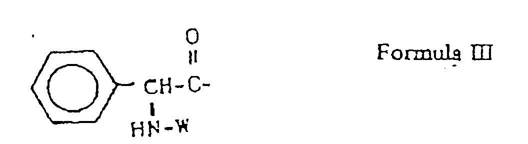 wherein W is hydrogen or t-butoxycarbonyl, and
wherein Y is a group represented by Formula IV or V 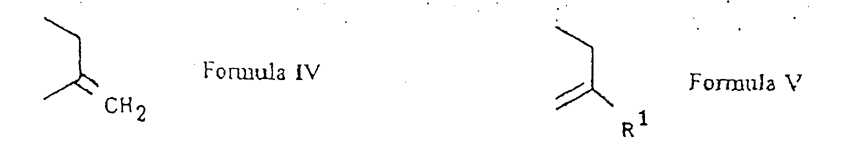 wherein R¹ is chloro, bromo, methyl, halomethyl, or methoxy, comprises treating the cephalosporin p-nitrobenzyl ester with a metal selected from the group consisting of iron, aluminum, and tin, and with hydrochloric acid in a mixture of water and a water miscible organic solvent.

According to a preferred process of this invention, a cephalosporin p-nitrobenzyl ester represented by Formula I is reacted with iron and hydrochloric acid in a mixture of water and an organic solvent at a temperature between 25-65°C, preferably at 30-50°C, to provide the corresponding free acid represented by Formula II.

For the best results in the process, iron in the form of a fine powder is preferred and is employed in an amount between 7 to 12 moles of iron per mole of ester; however, amounts of iron in excess of this mole ratio can also be employed.

In place of iron several other metals like aluminum, magnesium, and tin can also be used this invention. However, iron metal gives the best results.

Hydrochloric acid used in the process is present in a ratio of between 3 to 10 moles per mole of cephalosporin p-nitrobenzyl ester. Preferably, the mole ratio is about 2 to 5 moles of hydrochloric acid per mole of cephalosporin p-nitrobenzyl ester.

Organic solvents which can be employed in this process are commercially available and water miscible. These solvents include alcohols, such as methanol, ethanol or isopropanol, and ethers, such as tetrahydrofuran or dioxane. The preferred solvent in the process of this invention is methanol or tetrahydrofuran.

The reduction has been carried out in a water/organic solvent mixture wherein the water comprises 10 to 40 parts by volume and the organic solvent comprises from 60 to 90 parts by volume. The preferred volume ratio of water to organic solvent is 20 : 80 respectively.

The invention will now be described by reference to the following specific examples.

### EXAMPLE 1

### 7-AMINO-3-CHLORO-3-CEPHEM-4-CARBOXYLIC ACID

To a mixture of methanol (120ml) and water (25ml) p-nitrobenzyl 7-amino-3-chloro-3-cephem-4-carboxylate (10gm) was added. Iron powder (10gm) was charged at 30°C, followed by the addition of concentrated HCl (12.5ml) in a methanol (10ml) and water (2.5ml) mixture over a period of 15 minutes. The reaction mixture was stirred for 1 hour. After completion of the reaction, aq. KHCO₃ was added to bring the pH to 8.0, followed by the addition of activated carbon. After stirring for 10 minutes, the mixture was filtered. To the filtrate was added aq. HCl to bring the pH to 4.0. The product thus separated was filtered and dried to yield 4.6gm (80%) of 7-amino-3-chloro-3-cephem-4-carboxylic acid as a white solid. M.p. 210-212°C(d).
IR (KBr)cm⁻¹: 1785,1605,1520,1330,1075,850,805,775.
NMR (D₂O-NaHCO₃) δ:3.80 (dd, 2H, S-CH₂), 5.20 (d, 1H, S-CH), 5.50 (d, 1H, N-CH).

### EXAMPLE 2

### 7-AMINO-3-CHLORO-3-CEPHEM-4-CARBOXYLIC ACID

To a mixture of tetrahydrofuran (120ml) and water (25ml), p-nitrobenzyl 7-amino-3-chloro-3-cephem-4-carboxylate (10gm) was added. Iron powder (10gm) was charged at 30°C, followed by the addition of concentrated HCl (12.5ml) in a tetrahydrofuran (10ml) and water (2.5ml) mixture over a period of 15 minutes. The reaction mixture was stirred for 1 hour. After completion of the reaction, aq. KHCO₃ was added to bring the pH to 8.0, followed by the addition of activated carbon. After stirring for 10 minutes, the mixture was filtered. To the filtrate was added aq. HCl to bring the pH to 4.0. The product thus separated was filtered and dried. Yield 4.17gm (72.3%).

### EXAMPLE 3

### 7-AMINO-3-CHLORO-3-CEPHEM-4-CARBOXYLIC ACID

To a mixture of ethanol (240ml) and water (50ml), p-nitrobenzyl 7-amino-3-chloro-3-cephem-4-carboxylate (20gm) was added. Iron powder (20gm) was charged at 30°C, followed by the addition of concentrated HCl (25ml) in an ethanol (930ml) and water (5.0ml) mixture over a period of 15 minutes. The reaction mixture was stirred for 1 hour. After completion of the reaction, aq. KHCO₃ was added to bring the pH to 8.0, followed by the addition of activated carbon. After stirring for 10 minutes, the mixture was filtered. To the filtrate was added aq. HCl to bring the pH to 4.0. The product thus separated was filtered and dried to yield 8.42gm (73%) of white crystalline product.

### EXAMPLE 4

### 7-PHENOXYACETAMIDO-3-CHLORO-3-CEPHEM-4-CARBOXYLIC ACID

To a mixture of methanol (120ml) and water (25ml), p-nitrobenzyl 7-phenoxyacetamido-3-chloro-3-cephem-4-carboxylate (10gm) was added. Iron powder (10gm) was charged at 30°C, followed by the addition of concentrated HCl (25ml) in a methanol (10ml) and water (2.5ml) mixture over a period of 15 minutes. The reaction mixture was stirred for 1 hour and filtered. The solvent was removed under vacuum. The residue was taken up in ethyl acetate (200ml) and stirred with 10% aq. KHCO₃ soln. (100ml). The aqueous layer was separated, treated with 5% activated carbon, filtered and the pH was adjusted to 2.00 with conc. HCl. The product thus separated was filtered and dried to yield 3.0gm (41%) of the corresponding cephalosporanic acid. M.p. 168-170°C.
IR (KBr) cm⁻¹ : 3400, 1770, 1745, 1700, 1650, 1210, 750.
NMR (DMSO-d₆) δ:3.7 (dd, 2H, S-CH₂), 4.4 (s, 2H-OCH₂), 4.9 (d, 1H, S-CH), 5.5 (q, 1H, N-CH), 6.5-7.2 (m, 5H, Ar-H), 8.75 (d, 1H, NH).

### EXAMPLE 5

### 7-PHENYLACETAMIDO-3-CHLORO-3-CEPHEM-4-CARBOXYLIC ACID

The procedure described in Example 4 was repeated using p-nitrobenzyl 7-phenylacetamido-3-chloro-3-cephem-4-carboxylate as the starting material. The corresponding acid was obtained in 40.2% yield. M.p. 142-145°C.
IR (KBr) cm⁻¹ : 3260, 1755, 1635, 1515, 680.
NMR (DMSO-δ₆) δ: 3.2-4.2 (m, 4H, S-CH₂ & Ar-CH₂), 5.2 (d, 1HS-CH), 5.7 (q, 1H, N-CH), 7.3 (s, 5H, Ar-H), 9.1 (d, 1H, NH).

### EXAMPLE 6

### 7-(D-α-t-BUTOXYCARBOXAMIDOPHENYLACETAMIDO)-3-CHLORO-3-CEPHEM-4-CARBOXYLIC ACID

p-Nitrobenzyl 7-(d-α-t-butoxycarboxamidophenylacetamido)-3-chloro-3-cephem-4-carboxylate (10gm) was suspended in a mixture of methanol (120ml), THF (40ml) and water (25 ml). Iron powder (10gm) was charged at 30°C, followed by the addition of concentrated HCl (12.5ml) in a methanol (10ml) and water (2.5ml) mixture over a period of 15 minutes. The reaction mixture was stirred for 1 hour and filtered. The solvent was removed and the residue was taken up in ethyl acetate (150ml) and stirred with 10% aq. KHCO₃ solution (100ml). The aqueous layer was separated and treated with 5% activated carbon, filtered and acidified to pH 2.00 with conc. HCl. The solid separated was extracted in methylene chloride and on evaporation of methylene chloride the corresponding acid. 3.0gm (38%) was obtained. M.p. 112-115(d).
IR (KBr) cm⁻¹ : 3330, 2925, 1785, 1700, 1365, 1160, 690.
NMR (CDCl₃) δ:1.35 (s, 9H, CH₃), 3.9 (q, 2H,S-CH₂), 4.7 (d, 1H, S-CH), 5.3-6.1 (m, 2H, Ar-CH & N-CH), 7.0 (s, 5H, Ar-H).

### EXAMPLE 7

### 7-PHENOXYACETAMIDO-3-CHLORO-3-CEPHEM-4-CARBOXYLIC ACID

p-Nitrobenzyl 7-phenoxyacetamino-3-methylene-3-cephem-4-carboxylate-1-oxide (10gm) was suspended in a mixture of methanol (120ml), THF (40ml) and water (25 ml). Iron powder (10gm) was charged at 30°C, followed by the addition of concentrated HCl (12.5ml) in a methanol (10ml) and water (2.5ml) mixture over a period of 15 minutes. The reaction mixture was stirred for 1 hour. The reaction mixture was filtered to remove unreacted iron. The solvent was evaporated under vacuum and to the residue, ethyl acetate was added. The solid thus separated was filtered, washed with ethyl acetate and dried to yield 2.1gm (30%) of the corresponding cephalosporanic acid. M.p. 192-194°C.
IR (KBr) cm⁻¹ : 3540, 3350, 1775, 1670, 1595, 1435, 1280, 1245, 1160, 1100, 1015, 915, 855, 745.
NMR (DMSO-d₆) δ: 3.7 (dd, 2H, S-CH₂), 4.5(d, 2H, -OCH₂), 4.7-5.5 (m, 4H, exomethylene, N-CH & S-CH), 5.6 (q, 1H, HN-CH), 6.5(m, 5H, Ar-H), 7.9(d, 1H, NH).

### EXAMPLE-8

### 7-PHENLYACETAMIDO-3-CHLORO-3-CEPHEM-4-CARBOXYLIC ACID-1-OXIDE

Using the procedure described in Example 7, p-nitrobenzyl 7-phenlyacetamido-3-chloro-3-cephem-4-carboxylate-1-oxide was reacted with iron and HCl to obtain the corresponding acid in 28.4% yield. M.p. 185-191°C.
IR (KBr) cm⁻¹ : 3300, 1770, 1720, 1660, 1510, 1200, 1000, 720
NMR (DMSO-d₆) 8: 3.4 (d, 2H,S-CH₂), 3.6(d,2H,Ar-CH₂), 4.7-5.4 (m, 5H, N-CH, exomethylene (CH₂), S-CH & HOOC-CH), 6.9(s, 5H, Ar-H), 7.6(d, 1H,NH)

### EXAMPLE 9

### 7-AMINO-3-METHYL-3-CEPHEM-4-CARBOXYLIC ACID

To a mixture of ethanol (120ml) and water (25ml), p-nitrobenzyl 7-amino-3-methyl-3-cephem-4-carboxylate (10gm) was added. Iron powder (10gm) was charged at 30°C, followed by the addition of concentrated HCl (12.5ml) in a methanol (10ml) and water (2.5ml) mixture over a period of 15 minutes. The reaction mixture was stirred for 1 hour. After completion of the reaction, aq. KHCO₃ was added to bring the pH to 8.0, followed by the addition of activated carbon. After stirring 10 minutes, the mixture was filtered. To the filtrate was added aq. HCl to bring the pH to 4.0. The product thus separated was filtered and dried to yield 4.3gm (70.3%) of 7-amino-3-methyl-3-cephem-4-carboxylic acid as a white solid product. M.p. 217-219°C(d).
IR(KBr) cm⁻¹ 3150, 1800, 1650, 1625, 1525, 1420, 1350, 790.
NMR (D₂O-DCl) δ:2.1 (s, 3H,-CH₃), 5.41(d, 1H, S-CH), 5.86(m, 1H, N-CH).

### EXAMPLE 10

### 7-AMINO-3-CHLORO-3-CEPHEM-4-CARBOXYLIC ACID

p-Nitrobenzyl 7-amino-3-chloro-3-cephem-4-carboxylate (10gm) was suspended in a mixture of methanol (120ml) and water (25ml). Aluminum powder (10gm) was charged at 30°C, followed by the addition of concentrated HCl (12.5ml) in a methanol (10ml) and water (2.5ml) mixture over a period of 15 minutes. The reaction mixture was stirred for 1 hour. After completion of the reaction, aq. KHCO₃ was added to bring the pH to 8.0 followed by the addition of activated carbon. After stirring for 10 minutes, the reaction mixture was filtered. To the filtrate was added aq. HCl to bring the pH to 4.0. The product thus separated was filtered and dried. Yield: 3.9 gm (68%).

### EXAMPLE 11

### 7-AMINO-3-CHLORO-3-CEPHEM-4-CARBOXYLIC ACID

p-Nitrobenzyl 7-amino-3-chloro-3-cephem-4-carboxylate (10gm) was suspended in a mixture of methanol (120ml) and water (25ml). Tin powder was charged at 30°C, followed by the addition of concentrated HCl (25ml) in a methanol (20ml) and water (5ml) mixture over a period of 15 minutes. The reaction mixture was stirred for 1 hour. After completion of the reaction, aq. KHCO₃ was added to bring the pH to 8.0, followed by the addition of activated carbon. After stirring for 10 minutes, the mixture was filtered. To the filtrate was added aq. HCl to bring the pH to 4.0. The product thus separated was filtered and dried. Yield: 3.9g (68%).

While the invention has been described by reference to specific embodiments, this was for purposes of illustration only. Numerous alternative embodiments will be readily apparent to those skilled in the art and are considered to be within the scope of the invention.

## Claims

1. A process for converting a cephalosporin p-nitrobenzyl ester represented by Formula I 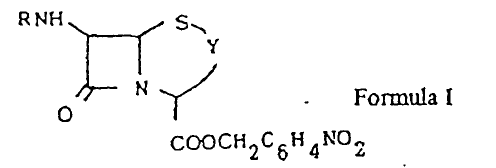 to a corresponding cephalosporin free acid represented by Formula II 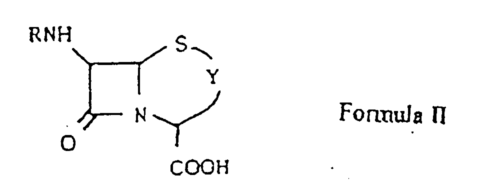 wherein R is hydrogen, phenylacetyl, phenoxyacetyl or a group represented by Formula III 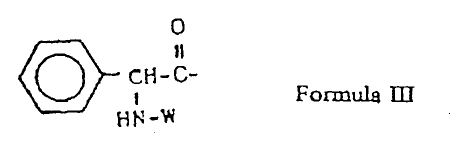 wherein W is hydrogen or t-butoxycarbonyl, and
wherein Y is a group represented by Formula IV or V 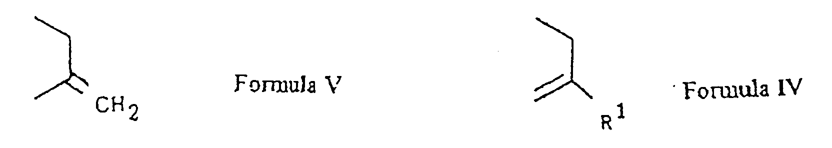 wherein R¹ is chloro, bromo, methyl, halomethyl, or methoxy, comprising treating the cephalosporin p-nitrobenzyl ester with a metal selected from the group consisting of iron,
aluminum, and tin, and with hydrochloric acid in a mixture of water and a water miscible organic solvent.

2. The process of claim 1 wherein the treatment is carried out at a temperature in the range of 25-65°C.

3. The process of claim 1 wherein the treatment is carried out at a temperature in the range of 30-50°C.

4. The process of claim 1 wherein the amount of hydrochloric acid is in the range of 3-10 moles per mole of said p-nitrobenzyl cephalosporin ester.

5. The process of claim 1 wherein the amount of hydrochloric acid is in the range of 2-5 moles per mole of said p-nitrobenzyl cephalosporin ester.

6. The process of claim 1 wherein the amount of said metal is in the range of 7-12 moles per mole of said p-nitrobenzyl cephalosporin ester.

7. The process of claim 1 wherein the amount of said metal is in the range of 8-10 moles per mole of said p-nitrobenzyl cephalosporin ester.

8. The process of claim 1 wherein said organic solvent is an alcohol, an ether, or a combination thereof.

9. The process of claim 8 wherein said alcohol is methanol, ethanol, or isopropanol.

10. The process of claim 8 wherein said ether is tetrahydrofuran or dioxane.

11. The process of claim 1 wherein said metal is iron.

12. The process of claim 11 wherein said iron is in the form of a fine powder

## Patentansprüche

1. Verfahren zur Umwandlung eines Cephalosporin-p-nitrobenzylesters der Formel I 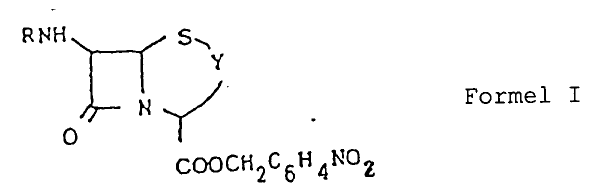 zu einer entsprechenden cephalosporinfreien Säure der Formel II, 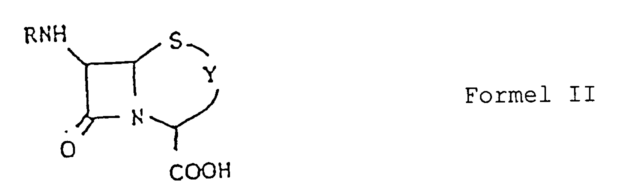 worin R Wasserstoff, Phenylacetyl, Phenoxyacetyl oder eine Gruppe der Formel III ist, 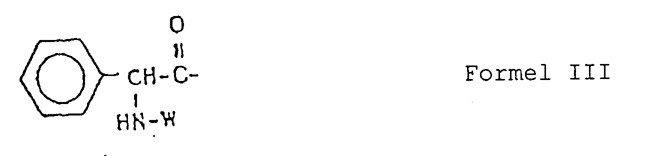 worin W Wasserstoff oder tert.-Butoxycarbonyl und Y eine Gruppe der Formel IV Oder V ist, 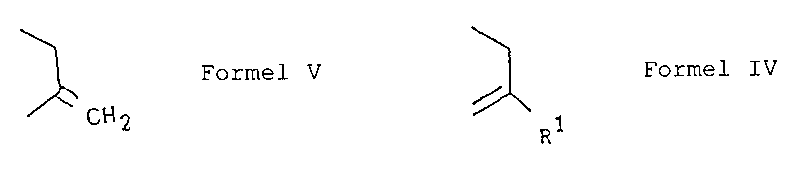 worin R¹ Chlor, Brom, Methyl, Halomethyl oder Methoxy ist, das ein Behandeln des Cephalosporin-p-nitrobenzylesters mit einem Metall, ausgewählt aus der Gruppe bestehend aus Eisen, Aluminium und Zinn sowie mit Salzsäure und einem mit Wasser mischbaren organischen Lösungsmittel umfaßt.

2. Verfahren nach Anspruch 1, worin die Behandlung bei einer Temperatur in einem Bereich von 25 bis 65°C erfolgt.

3. Verfahren nach Anspruch 1, worin die Behandlung bei einer Temperatur in einem Bereich von 30 bis 50°C erfolgt.

4. Verfahren nach Anspruch 1, worin die Menge der Salzsäure in einem Bereich von 3 bis 10 Mol pro Mol p-Nitrobenzylcephalosporinester liegt.

5. Verfahren nach Anspruch 1, worin die Menge der Salzsäure in einem Bereich von 2 bis 5 Mol pro Mol p-Nitrobenzylcephalosporinester liegt.

6. Verfahren nach Anspruch 1, worin die Menge des Metalls in einem Bereich von 7 bis 12 Mol pro Mol p-Nitrobenzylcephalosporinester liegt.

7. Verfahren nach Anspruch 1, worin die Menge des Metalls in einem Bereich von 8 bis 10 Mol pro Mol p-Nitrobenzylcephalosporinester liegt.

8. Verfahren nach Anspruch 1, worin das organische Lösungsmittel ein Alkohol, Ether oder eine Mischung hieraus ist.

9. Verfahren nach Anspruch 8, worin der Alkohol Methanol, Ethanol oder Isopropanol ist.

10. Verfahren nach Anspruch 8, worin der Ether Tetrahydrofuran oder Dioxan ist.

11. Verfahren nach Anspruch 1, worin das Metall Eisen ist.

12. Verfahren nach Anspruch 11, worin das Eisen in Form eines feinen Pulvers vorliegt.

## Revendications

1. Procédé de conversion d'un ester p-nitrobenzylique de cephalosporine de formule I 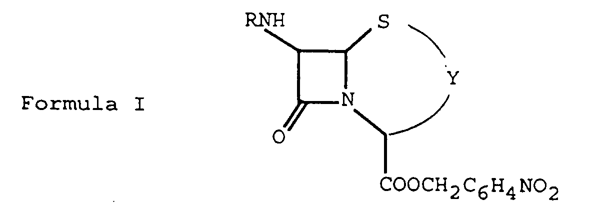 en une céphalosporine correspondante sous forme d'acide libre représentée par la formule II 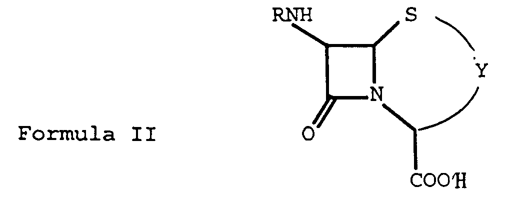 dans laquelle R est hydrogène, phénylacétyle, phénoxyacétyle ou un groupe représenté par la formule III 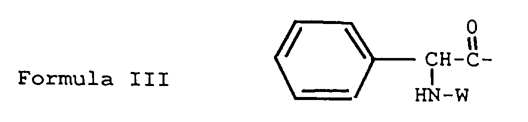 dans laquelle W est hydrogène ou t-butoxycarbonyle, et
dans laquelle Y est un groupe représenté par la formule IV ou V 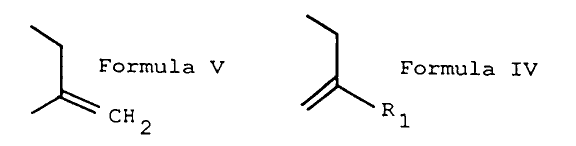 dans lesquelles R¹ est chloro, bromo, méthyle, halométhyle ou méthoxy,
comprenant le traitement de l'ester p-nitrobenzylique avec un métal sélectionné dans le groupe constitué par le fer, l'aluminium et l'étain, et avec l'acide chlorhydrique dans un mélange d'eau et de solvant organique miscible à l'eau.

2. Procédé selon la revendication 1 dans lequel le traitement est mené à une température comprise entre 25 et 65°C.

3. Procédé selon la revendication 1 dans lequel le traitement est mené à une température comprise entre 30 et 50°C.

4. Procédé selon la revendication 1 dans lequel la quantité d'acide chlorhydrique varie de 3 à 10 moles par mole dudit ester p-nitrobenzylique de céphalosporine.

5. Procédé selon la revendication 1 dans lequel la quantité d'acide chlorhydrique varie de 2 à 5 moles par mole dudit ester p-nitrobenzylique de céphalosporine.

6. Procédé selon la revendication 1 dans lequel la quantité dudit métal varie de 7 à 12 moles par mole dudit ester p-nitrobenzylique de céphalosporine.

7. Procédé selon la revendication 1 dans lequel la quantité dudit métal varie de 8 à 10 moles par mole dudit ester p-nitrobenzylique de céphalosporine.

8. Procédé selon la revendication 1 dans lequel ledit solvant organique est un alcool, un éther ou un mélange de ceux-ci.

9. Procédé selon la revendication 8 dans lequel l'alcool est le méthanol, l'éthanol ou l'isopropanol.

10. Procédé de la revendication 8 dans lequel ledit éther est le tétrahydrofuranne ou le dioxanne.

11. Procédé selon la revendication 1 dans lequel ledit métal est le fer.

12. Procédé selon la revendication 11 dans lequel ledit fer est sous la forme d'une poudre fine.
